# EUROPEAN PATENT APPLICATION

(11) **EP 2 172 565 A1**
(43) Date of publication of application: **07.04.2010**
(21) Application number: 08164974.1
(22) Date of filing: 24.09.2008
(51) Int. Cl.: C12Q 1/68

(54) **Method of identifying and/or differentiating cyanophyta**

(71) Applicant: Cyano Biotech GmbH, 12489 Berlin (DE)
(72) Inventor: Kramer, Dan, 12489 Berlin (DE); Meixner, Martin, 15562 Rüdersdorf (DE); Lindner, Christina, 10247 Berlin (DE)
(74) Representative: Vossius & Partner

(57) **Abstract**

The invention relates to a method of identifying and/or differentiating cyanophyta comprising the steps of, (a) enzymatically digesting at least a first genomic DNA from a cyanophyta isolate to be identified or differentiated, with at least two different restriction enzymes, into restriction fragments, wherein a first restriction enzyme is a four cutter and a second enzyme is a six cutter, (b) ligating double stranded nucleic acid oligonucleotide adaptor fragments to the nucleic acid ends of the restriction fragments created in step (a), (c) pre-amplifying the fragments using pre-amplification primers which are substantially complementary to at least part of the restriction sites generated in step (a) and substantially complementary to at least part of the double stranded nucleic acid oligonucleotide adaptor fragments ligated in step (b), (d) selectively amplifying the fragments generated after step (c) by using (i) least one pre-amplifying primer and (ii) at least one selection primer which is substantially complementary to at least part of the restriction sites generated in step (a) and substantially complementary to at least part of the double stranded nucleic acid oligonucleotide adaptor fragments ligated in step (b) and which comprises at least two additional nucleotides at its 3'-OH. The invention also relates to kits.

## Description

### INTRODUCTION

Cyanobacteria, also known as blue-green algae, blue-green bacteria or Cyanophyta, is a phylum of bacteria that obtain their energy through photosynthesis. They are a significant component of the marine nitrogen cycle and an important primary producer in many areas of the ocean, but are also found in fresh water, in symbiosis or on land. They are able to occupy nearly every biotope (environment) on earth.

Stromatolites of fossilized oxygen-producing cyanobacteria have been found from 2.8 billion years ago. The ability of cyanobacteria to perform oxygenic photosynthesis is thought to have converted the early reducing atmosphere into an oxidizing one, which dramatically changed the life forms on Earth and provoked an explosion of biodiversity. Chloroplasts in plants and eukaryotic algae have evolved from cyanobacteria via endosymbiosis.

The cyanobacteria were traditionally classified by morphology into five sections, referred to by the numerals I-V. The first three - *Chroococcales, Pleurocapsales,* and *Oscillatoriales -* are not supported by phylogenetic studies. However, the latter two - *Nostocales* and *Stigonematales -* are monophyletic, and make up the heterocystous cyanobacteria. The members of *Chroococales* are unicellular and usually aggregated in colonies. The classic taxonomic criterion has been the cell morphology and the plane of cell division. In *Pleurocapsales,* the cells have the ability to form internal spores (baeocytes). The rest of the section includes filamentous species. In *Oscillatorialles,* the cells are uniseriately arranged and do not form specialized cells (akinets and heterocysts). In *Nostocalles* and *Stigonematalles* the cells have the ability to develop heterocysts under certain conditions. *Stigonematales,* unlike *Nostocalles* include species with truly branched trichome.

Certain cyanobacteria produce cyanotoxins like anatoxin-a, anatoxin-as, aplysiatoxin, cylindrospermopsin, domoic acid, microcystin LR, nodularin R (from Nadularia), or saxitoxin. Sometimes a mass-reproduction of cyanobacteria results in algal blooms.

The unicellular cyanobacterium *Synechocystis sp. PCC6803* was the third prokaryote and first photosynthetic organism whose genome was completely sequenced. To date approx. 50 cyanobacterial genomes are completely sequenced.

At least one secondary metabolite, cyanovirin, has shown to possess anti-HIV activity.

Some cyanobacteria are sold as food, notably *Aphanizomenon* flos-aquae and *Arthrospira platensis* (Spirulina). It has been suggested that they could be a much more substantial part of human food supplies, as a kind of superfood.

Along with algae, some hydrogen producing cyanobacteria are being considered as an alternative energy source.

Cyanabacfieriai peptides (cyanopeptides) as well as polyketides are among the most ubiquitously found potentially hazardous natural products in surface waters used by humans. Though these substances are natural in origin, eutrophication (*i*.*e*. excessive loading with fertilising nutrients) has caused massive cyanobacterial proliferation throughout Europe. Thus, cyanopeptides now occur with unnatural frequency and concentration. A large group among the diverse cyanopeptides are the oligopeptides (peptides with a molecular weight of <2KD). But while specific cyanopeptides - e.g. microcystins and nodularins - are well studied and recognised as being causative for many animal poisonings and human illness, a substantial and increasing body of evidence points toward a decisive role of other potentially toxic cyanopeptides in the causation of both acute and chronic human illnesses.

Freshwater and marine cyanobacteria are known to produce a variety of bioactive compounds, among them potent hepatotoxins and neurotoxins. Many of the toxic species of cyanobacteria tend to massive proliferation in eutrophicated water bodies and thus have been the cause for considerable hazards for animal and human health. One of the most widespread bloom- forming cyanobacteria is the genus *Microcystis,* a well-known producer of the hepatotoxic peptide microcystin. Microcystins are a group of closely related cyclic heptapeptides sharing the common structure. So far, more than 80 derivatives of microcystins have been identified, varying largely by the degree of methylation, peptide sequence, and toxicity.

The traditional botanical code describes the genus *Microcystis* as a coccal, unicellular cyanobacterium that grows as mucilaginous colonies of irregularly arranged cells (under natural conditions, while strain cultures usually grow as single cells). According to this tradition, morphological criteria such as size of the individual cells, colony morphology, and mucilage characteristics are used for species delimitation within Microcystis (*i*.*e*., morphospecies). Microcystin-producing strains as well as strains that do not synthesize microcystin have been reported for all species within the genus Microcystis. However, whereas most field samples and strains of *Microcystis aeruginosa* and *Microcystis viridis* studied to date were found to contain microcystins, strains of M. *wesenbergii, M. novaceckii, and* M. *iclathyoblabe* have only sporadically been reported to contain microcystins.

Beside microcystins, various other linear and cyclic oligopeptides such as anabaenopeptins, aeruginosins, microginins and cyanopeptolins are found within the genus *Microcystis* (Namikoshi, M., and K. L. Rinehart. 1996. Bioactive compounds produced by cyanobacteria. J. Ind. Microbiol. 17:373-384.).

Similar to microcystins, these peptides possess unusual amino acids like 3-amino-6-hydroxy- 2-piperidone (Ahp) in cyanopeptolins, 2-carboxy-6-hydroxyoctahydroindol (Choi) in aeruginosin-type molecules or 3-amino-2 hydroxy-decanoic acid (Ahda) in microginins and numerous structural variants also exist within these groups. These peptides show diverse bioactivities, frequently protease inhibition (Namikoshi, M., and K. L. Rinehart. 1996. Bioactive compounds produced by cyanobacteria. J. Ind. Microbiol. 17:373-384).

The occurrence of both microcystins and other oligopeptides such as anabaenopeptins, microginins and cyanopeptolins in natural Microcystis populations was recently demonstrated. It is well known that the species and genotype composition in natural *Microcystis* populations is heterogeneous, and both microcystin- and non-microcystin-confiaining strains have been isolated from the same sample. Just as strains producing microginin and strains not producing microginin have been found. These results suggest a cnnsiderable diversity of genotypes with different oligopeptide patterns in natural Microcystis populations.

Chemical screening of cyanobacterial samples (both from field samples and from culture strains) has demonstrated a wide variety of substances: e.g. an almost monospecific bloom of *Planktothrix agardhii* contained as many as 255 different substances, most of which were oligopeptides.

In fact PEPCY a research project supported by the European Commission concluded that present information shows that one species or "morphotype" (*i*.*e*. individuals with the same morphological characteristics) may comprise a range of genotypes that encode for different "chemotypes" (*i*.*e*. morphofoglcally indistinguishable individuals containing different cyanopeptides).

Hence, cyanobacteria are group of microorganisms with a very broad diversity not only on the genus level but especially on the level of species and strains. This feature is typical for cyanobacteria and reflects both their ecology and molecular biology. During the course of evolution cyanobacteria have adapted to almost every ecological niche, including the most extreme ones. They can be found in freshwater lakes and oceans, as well as in deserts, hot and acidic springs, and even in the arctic ice. The adaptability of cyanobacteria is based on their enormous diversity in species and strains which is reflected by their genomes. Strains of one and the same species often significantly differ in their physiological features, e.g. they synthesize diverse natural products. The corresponding biosynthetic gene clusters were passed on by horizontal gene transfer from one strain or even genus to another one. It is also known that cyanobacterial genomes are frequently influenced by transposons as well as by phages.

The inventors could show that it is not possible to differentiate strains belonging to the same species by DNA sequencing of established taxonomic markers like 16 S rDNA, ITS region (ITS - internal transcribed spacer) or PC gene (phycocyanin gene; used especially for cyanobacteria). Also further differentiation methods (e.g. morphological description by microscopically analysis, protein fingerprints by mass spectroscopy) did not result in a differentiation of strains on species level.

The problem at hand is that there are no methods available for quickly identifying and differentiating cyanobacterial sections and/or strains. The industrial use of these bacteria makes it necessary to be able to quickly identify the strain. This is often necessary for quality or health reasons.

Therefore it is the objective of the present invention to provide for a method of identifying and/or differentiating cyanophyta.

### BRIEF DESCRIPTION OF THE INVENTION

The invention relates to a method of identifying and/or differentiating cyanophyta comprising the steps of,
(a) enzymatically digesting at least a first genomic DNA from a cyanophyta isolate to be identified or differentiated, with at least two different restriction enzymes, into restriction fragments, wherein a first restriction enzyme is a four cutter and a second enzyme is a six cutter,
(b) ligating double stranded nucleic acid oligonucleotide adaptor fragments to the nucleic acid ends of the restriction fragments created in step,
(c) pre-amplifying the fragments using pre-amplification primers which are substantially complementary to at least part of the restriction sites generated in step (a) and substantially complementary to at least part of the double stranded nucleic acid oligonucleotide adaptor fragments ligated in step (b),
(d) selectively amplifying the fragments generated after step (c) by using
   (i) least one pre-amplifying primer and
   (ii) at least one selection primer which is substantially complementary to at least part of the restriction sites generated in step (a) and substantially complementary to at least part of the double stranded nucleic acid oligonucleotide adaptor fragments ligated in step (b) and which comprises at least two additional nucleotides at its 3'-OH.

Cyanophyta in accordance with the present invention are, representative members out of all cyanobacteria sections I to V.

Herein, a restriction endonuclease or restriction enzyme is an enzyme that recognizes a specific base sequence (target site) in a double-stranded DNA molecule, and will cleave both strands of the DNA molecule at every target site.

Herein restriction fragments are DNA molecules produced by digestion with one or more restriction endonucleases. Any given genome will be digested by a set of two particular restriction endonucleases into a discrete set of restriction fragments. The DNA fragments that result from restriction endonuclease cleavage are separated and detected by size determination methods.

Restriction fragment length polymorphisms (RFLPs) in the genomic DNA of two closely related organisms, for example, will exhibit differences in their nucleotide sequence composition at many sites. When these differences occur in the target site for a restriction endonuclease, the modified target site will not be cleaved at that point. Likewise, a nucleotide sequence variation may introduce a novel target site where none exists in the other organism, causing the DNA to be cut by the restriction enzyme at that point. Alternatively, insertions or deletions of nucleotides occurring in one organism between two target sites for a restriction endonuclease will modify the distance between those target sites. Because of this, digestion of the two organism's DNA will produce restriction fragments having different lengths. A polymorphism in the length of restriction fragments produced by digestion of the DNA of the two organisms will result.

Herein, ligation is the enzymatic reaction catalyzed by the enzyme ligase in which two double-stranded DNA molecules are covalently joined together. In general, both DNA strands are covalently joined together, but it is also possible to prevent the ligation of one of the two strands, through chemical or enzymatic modification of one of the ends. In that case the covalent joining will occur in only one of the two DNA strands.

Herein, adaptors or adaptor fragments are short double-stranded DNA molecules, with a limited number of base pairs, e.g. 10 to 50 base pairs long, which are designed in such a way that they can be ligated to the ends of restriction fragments. Adaptors are composed of two synthetic oligonucleofiides which have nucleotide sequences which are in part complementary to each other. When mixing the two synthetic oligonucleotides, they will form a double-stranded structure in solution under appropriate conditions. One of the ends of the adaptor molecule is designed so that it can be ligated to the end of a restriction fragment, the other end is designed so that it cannot be ligated.

Polymerase Chain Reaction (PCR) herein is the enzymatic reaction in which DNA fragments are synthesized from a substrate DNA in vitro is referred to as PCR. The reaction involves the use of two synthetic oligonucleotides, which are complementary to nucleotide sequences in DNA molecules which are separated by a short distance of a few hundred to a few thousand base pairs, and the use of a thermostable DNA polymerase. The chain reaction consists for example of a series of 10 to 30 cycles. In each cycle the substrate DNA is first denaturated at high temperature. After cooling down the synthetic oligonucleotides which are present in vast excess will form double-stranded structures with the substrate DNA molecules in solution at specific sites on the substrate DNA molecule that have complementary nucleotide sequences. The oligonucleotide-substrate DNA complexes will then serve as initiation sites for the DNA synthesis reaction catalyzed by the DNA polymerase, resulting in the synthesis of a new DNA strand complementary to the substrate DNA strand.
Herein, the terms DNA amplification and PCR will be used to denote the synthesis of double-stranded DNA molecules *in vitro* using Polymerase Chain Reaction (PCR). The products of the PCR reaction will be referred to as amplified DNA fragments or PCR products.

Herein primer in general refers to a single stranded DNA molecule which can prime the synthesis of DNA. DNA polymerase cannot synthesize DNA de *novo* without primers: it can only extend an existing DNA strand in a reaction in which the complementary strand is used as a template to direct the order of nucleotides to be assembled. We will refer to the synthetic oligonucleotide molecules which are used in the PCR reaction as primers.

Oligonucleotide primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981), which is hereby incorporated by reference. One method for synthesizing oligonucleotides on a modified solid support is described in U.S. Pat. No. 4,458,006, which is hereby incorporated by reference. It is also possible to use a primer which has been isolated from a biological source (such as a restriction endonuclease digest). Preferred primers have a length of from about 15-100, more preferably about 20-50, most preferably about 20-40 bases.

Herein, a pre-amplifying primer is a short sequence of nucleotides (single stranded oligonucleotide) at least partially complementary to the adaptor sequence which is used in the first PCR based amplification step after ligation of the adaptors. This step is used for the multiplication of the target DNA fragments.

Herein, a selection or selective primer is a single stranded oligonucleotide at least partially complementary to the adaptor and preamplification primer sequence and fully or partially degenerated in 1 to 4 positions at the 3 terminus of the oligonucleotide which is used in the second PCR based amplification step.

Specifically, this invention provides means for the identification of either individual genomic restriction fragments or of sets of genomic restriction fragments from cyanophyta, which are either individually genetically linked to one or more particular traits or that collectively provide a fingerprint of the genome that can be used to identify the cyanophyta organism, the variety or the strain.

The general method of the invention for production and for identification of restriction fragments in cyanophyta involves the use of restriction endonucleases, ligation of synthetic oligonucleotides to the restriction fragments, and PCR amplification of restriction fragments. Restriction endonucleases cleave genomic DNA molecules at specific sites, target sites, thereby generating restriction fragments.

PCR amplification of restriction fragments no matter whether one knows the nucleotidic sequence of the ends of the restriction fragments or not, can be achieved according to the invention, by first ligating synthetic oligonucleotides (adaptors) to the ends of restriction fragments, thus providing each restriction fragment with two common tags which will serve as a anchor base for the primers used in PCR amplification.
Typically, restriction enzymes produce either flush ends, in which the terminal nucleotides of both strands are base paired, or staggered ends in which one of the two strands protrudes to give a short single strand extension. In the case of restriction fragments with flush ends, adaptors are used with one flush end. In the case of restriction fragments with staggered ends adaptors are used which have a single stranded extension which is complementary to the single stranded extension of the restriction fragment. Consequently, for each type of restriction fragment specific adaptors are used, which differ only in one of the ends so as to allow the adaptor to be ligated to the restriction fragment. Typically, the adaptors used are composed of two synthetic oligonucleotides which are in part complementary to each other, and which are usually approximately 10 to 30 nucleotides long, preferably 12 to 22 nucleotides long and which form double-stranded structures when mixed together in solution. Using the enzyme ligase the adaptors are ligated to the mixture of restriction fragments. When using a large molar excess of adaptors over restriction fragments one ensures that all restriction fragments will end up carrying adaptors at both ends. Restriction fragments prepared with this method will be referred to as tagged restriction fragments and the method will be further referred to as restriction fragment tagging.

As used herein, a kit is a packaged combination optionally including instructions for use of the combination and/or other reactions and components for such use.

### DETAILED DESCRIPTION OF THE INVENTION

The inventors have astonishingly found that it is very difficult to type Cyanophyta. 16 S rRNA sequencing is tedious. It turns out that the traditional Selective Restriction Fragment Amplification Fingerprinting (AFLP) method using standard protocols or commercial kits only resulted in a small number of fragments, too small for successful discrimination of closely related species or strains within the cyanophyta. Amplified fragment length polymorphism PCR (or AFLP-PCR or just AFLP) is a PCR-based tool used in genetics research, DNA fingerprinting, and in the practice of genetic engineering. Developed in the early 1990's AFLP uses restriction enzymes to cut genomic DNA, followed by ligation of adaptors to the sticky ends of the restriction fragments. A subset of the restriction fragments are then amplified using primers complementary to the adaptor and part of the restriction site fragments. The amplified fragments are visualized on denaturing polyacrylamide gels either through autoradiography or fluorescence methodologies.

In detail, the procedure of this technique is divided into three steps:
a) Digestion of total cellular DNA with one or more restriction enzymes and ligation of restriction half-site specific adaptors to all restriction fragments.
b) Selective amplification of some of these fragments with two PCR primers that have corresponding adaptor and restriction site specific sequences.
c) Electrophoretic separation of amplicons on a gel matrix, followed by visualisation of the band pattern.

In an attempt to create a method of identifying and/or differentiating cyanophyta the inventors identified some aspects of AFLP that seem to apply only or primarily to the genome of cyanophyta:
The invention relates to a method of identifying and/or differentiating cyanophyta comprising the steps of,
   (a) enzymatically digesting at least a first genomic DNA from a cyanophyta isolate to be identified or differentiated, with at least two different restriction enzymes, into restriction fragments, wherein a first restriction enzyme is a four cutter and a second enzyme is a six cutter,
   (b) ligating double stranded nucleic acid oligonucleotide adaptor fragments to the nucleic acid ends of the restriction fragments created in step,
   (c) preamplifying the fragments using preamplification primers which are substantially complementary to at least part of the restriction sites generated in step (a) and substantially complementary to at least part of the double stranded nucleic acid oligonucleotide adaptor fragments ligated in step (b),
   (d) selectively amplifying the fragments generated after step (c) by using
      (i) least one preamplification primer and
      (ii) at least one selective primer which is substantially complementary to at least part of the restriction sites generated in step (a) and substantially complementary to at least part of the double stranded nucleic acid oligonucleotide adaptor fragments ligated in step (b) and which comprises at least two additional nucleotides at its 3'-OH.

It turns out that the preamplification step is important as well as the selective amplification step. However, for the first time an MseI-based preamplification primer was combined together with one ore more EcoRI-based selective primer(s) in the selective amplification reaction.

Preferably, the step of selective amplification is performed using two or more different selective primers, (a) first selective primer which is substantially complementary to at least part of the six cutter restriction sites generated in step (a) and substantially complementary to at least part of the double stranded nucleic acid oligonucleotide adaptor fragments ligated in step (b), but comprises two additional nucleotides at the 3'-OH end and, (b) a second selection primers which is substantially complementary to at least part of the six cutter restriction sites generated in step (a) and substantially complementary to at least part of the double stranded nucleic acid oligonucleotide adaptor fragments ligated in step (b), but comprises two additional nucleotides at the 3'-OH end, wherein the first, second or further selection primer do not have the same sequence and preferably carry a different fluorescent label.

It is preferred that the restriction enzymes used in step (a) are EcoRI and MseI. If EcoRI and MseI are used in the restriction step, it is preferred that the ratio of the amount of enzymes EcoRI to MseI is 1 to 2.

After inactivation of the restriction enzymes a ligation reaction between the adaptor molecules and the restriction fragments is performed.

Preferably the double stranded nucleic acid oligonucleotide adaptor molecules consist of the following nucleic acid molecules:
a. EcoRI - adaptor (+) 5'-CTC GTA GAC TGC GTA CC-3' (SEQ ID NO. 1) and EcoRI - adaptor (-) 5'- AAT TGG TAC GCA GTC TAC -3' (SEQ ID NO. 2) and
b. MseI - adaptor (+) 5'- GAC GAT GAG TCC TGA G -3' (SEQ ID NO. 3) and MseI - adaptor (-) 5'- TAC TCA GGA CTC AT -3' (SEQ ID NO. 4).

In accordance with the adaptor molecules the preamplification primers have the following sequences, EcoRI - pre-amplification primer 5'- GACTGCGTACCAATTC-3' (SEQ ID NO. 5) and MseI - pre-amplification primer 5'- GATGAGTCCTGAGTAA-3' (SEQ ID NO. 6)

It is preferred that the molarity of the EcoRI primer in the reaction is between 0.01 µM and 0.25 µM and the molarity of the MseI primer in the reaction is between 0.06 µM and 1.5 µM.

Further more a 5 to 10 fold excess of the MseI primer to the EcoRI primer is suggested. However, such ratios may be from 1:1 (EcoRI primer:MseI primer) to 1:50 (EcoRI primer:MseI primer), preferably from 1:1 (EcoRI primer:MseI primer) to 1:20 (EcoRI primer:MseI primer) and preferably from 1:1 (EcoRI primer:MseI primer) to 1:10 (EcoRI primer:MseI primer). It is in general also possible but not preferred that the MseI primer is in excess. After preamplification a selection-amplification step is carried out. By this application only a part of fragments off the pre-amplification step are amplified. This is to reduce the number of fragments to an analyzable value.

Preferably, at least a combination of two ore more of the selection primers are selected from the following group of primers and wherein these have the following nucleotides at their respective 3'-OH terminal end:
EcoRI-Primer-CA-3'
EcoRI-Primer-GT-3'
EcoRI-Primer-TA-3'
EcoRI-Primer-TT-3'

In the selective amplfication step preferably two selective primers are used and one MseI preamplification primer is used.

Most preferably the selective primers that may by used have the following sequences:
EcoRI-Primer- 5'- GACTGCGTACCAATTCCA - 3' (SEQ 1D NO. 7)
EcoRI-Primer- 5' -.GACTGCGTACCAATTCGT - 3'(SEQ ID NO. 8)
EcoRI-Primer- 5'- GACTGCGTACCAATTCTA - 3'(SEQ ID NO. 9)
EcoRI-Primer-5'- GACTGCGTACCAATTCTT - 3'(SEQ ID NO. 10)

The method according to the invention may be practiced using various detection techniques. Preferably, the rare cutter selection primers are labelled. Preferably the label is a fluorescent dye.
In the context of the present invention, fluorescent dyes may for example be FAM (5-or 6-carboxyfluorescein), VIC, NED, Fluorescein, Fluoresceinisothiocyanafie (FITC), IRD-700/800, Cyanine dyes, auch as CY3, CY5, CY3.5, CY5.5, Cy7, Xanthen, 6-Carbaxy-2`,4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine,Rhodamine Green, Rhodamine Red, Rhodamine 110,
BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

The invention also relates to a kit comprising at least 2 pre-amplifying primers, 2 or 3 selection primers and a sample from Cyanophyta. Ideally such a kit would provide not just a sample but also isolated DNA from Cyanophyta. The kit may also have no sample but only DNA from Cyanophyta.

### FIGURE CAPTIONS

Fig. 1 shows the ligation of the adapter molecules to the restriction sites of the fragment
Fig. 2 shows the principle of the pre-amplification; the figure demonstrated the attachment of the EcoRI- and MseI-pre-primers to the fragment
Fig. 3 shows the results of testing different annealing temperatures to demonstrate the robustness and the reproducibility of the method: lane 1-4 disclose fragments from non-cyanophyta bacteria after pre-amplification; lane 5-8 disclose fragments from cyanophyta. The PCR was done in a gradient cycle with a non-linear gradient. The marker is 100, 200, 300, 400, 500, 600, 700, 800, 900, 1000, 1200, 1500, 2000 and 3000 bp.
Fig. 4A and 4B show the chromatograms for 4 different Synechocystis sp. PCC 6803 strains generated from the program "GeneMapper v4.0" (Applied Biosystems, CA, USA).

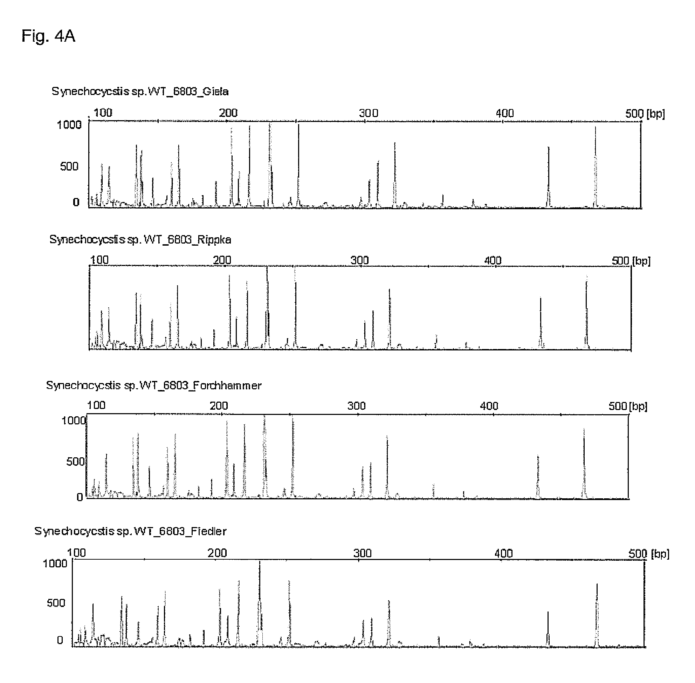

Fig. 4A shows the fragments, which are produced by the 6-FAM-marked selection primer.

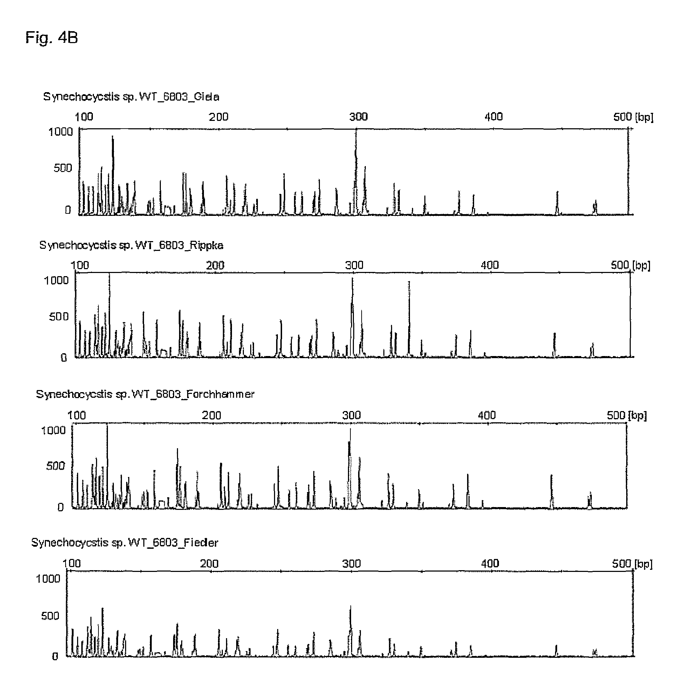

Fig. 4B shows the fragments, which are produced by the HEX-marked selection primer.

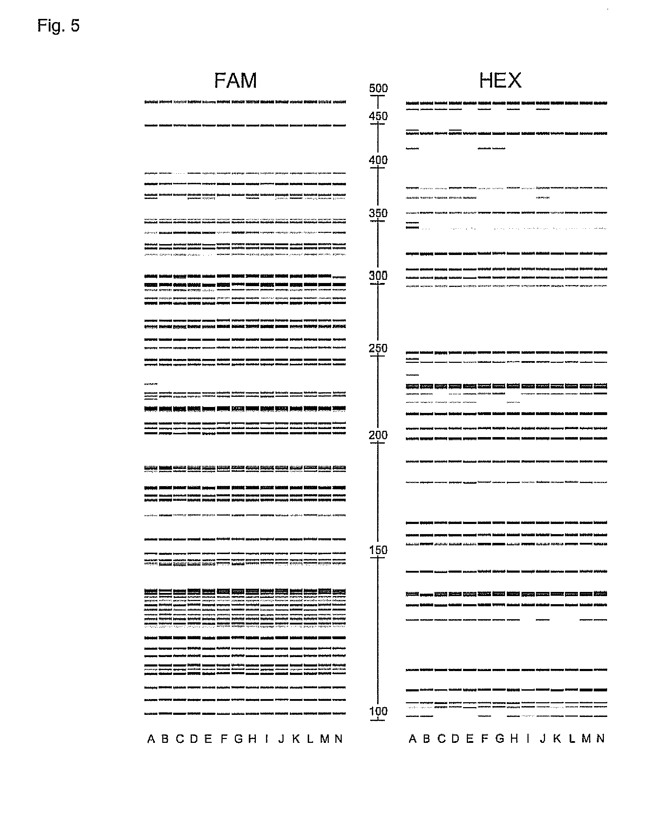

Fig. 5 shows the banding patterns for 14 different Synechocystis sp. PCC 6803 strains (Lane A - N) generated from the program "GelCompae" (Applied Maths, Sint-Martens-Latem, Belgium). The left block shows the banding patterns generated by the chromatogram data of the 6-FAM-labeled fragments. The right block shows the banding patterns generated by the chromatogram data of the HEX-labeled fragments.

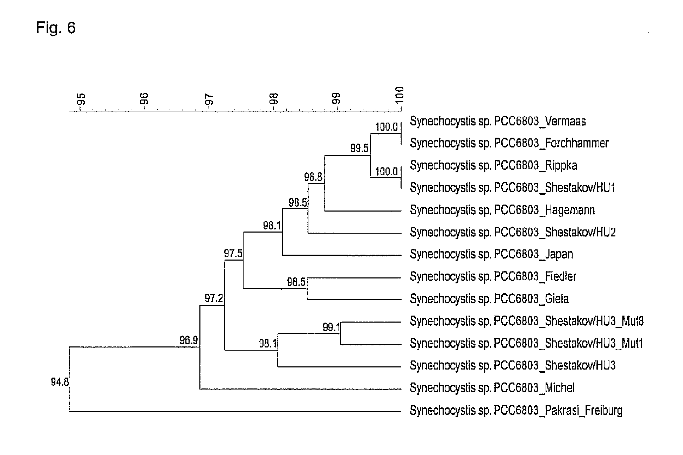

Fig. 6 discloses the dendrogram computed from the bending patterns of Fig. 5. The similarities [%] between the different strains are shown at the knot points of the dendrogram.

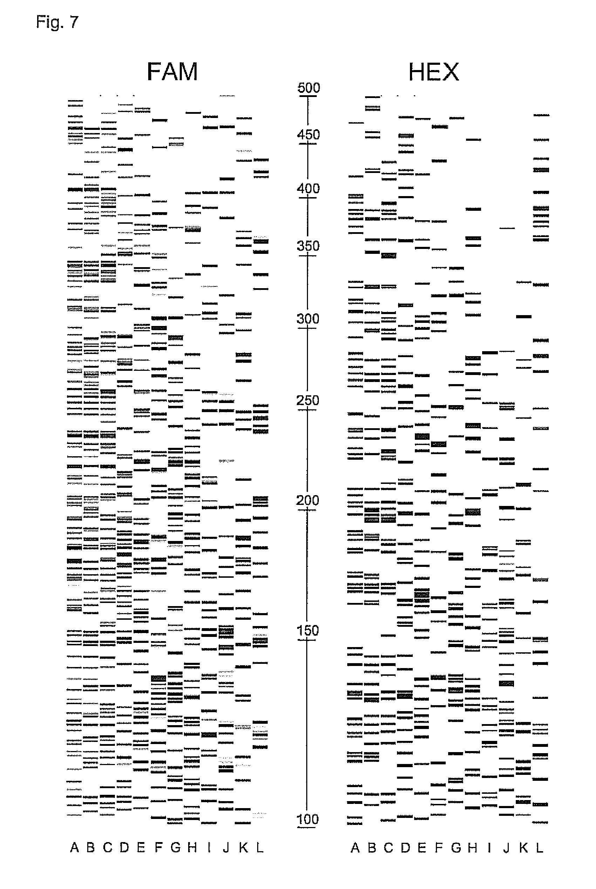

Fig. 7 shows the banding patterns for 12 different species (Lane A - C: Leptolyngbya, D: Trichormus, E: Pseudoanabena, F: Synechocystis, G: Nostoc, H: Limnothrix, I - L: Prochlorococcus) generated from the program "GelComparll". The left block shows the banding patterns generated by the chromatogram data of the 6-FAM-labeled fragments. The right block shows the banding patterns generated by the chromatogram data of the HEX-labefed fragments.

### EXAMPLES

Cyano bacteria

### Isolation and cultivation of Cyanophyta

The isolation of the cyanobacteria occurred making use of sediment from water that was taken up the Lower Oder River. Cultivation was done with Agar plates using BG 11 minimal media (ATCC, Medias 616). See Table 1 below.

BG 11 minimal media for cultivating cyanophyta.

**Table 1: BG 11 minimal media for cyanophyta**

| **BG-11 Media for Cyanophyta** | |
|---|---|
| | |
| NaNO₃ | 1,5 g |
| K₂HPO₄ | 0,04 g |
| MgSO₄ ·7 H₂O | 0,075 g |
| CaCl₂ · 7 H₂O | 0,036 g |
| Citric acid | 0,006 g |
| Ferric ammonium citrate | 0,006 g |
| EDTA | 0,001 g |
| NaCO₃ | 0,05 g |
| Micronutrient solution | 1,0 ml |
| Agar | 10,0 g |
| Dist. water | 11000 ml |

Additionally, upon sterilisation micronutrient solution A5 (Trace Metal Mix A5, ATCC) was added via sterile filter. See table 2 below.

**Table 2: micronutrient solution A5 for cultivating cyanobacteria**

| **Micronutrient sotution A5** | |
|---|---|
| | |
| H₃BO₃ | 2,86 g |
| MnCl₂ · 4 H₂O | 1,81 g |
| ZnSO₄ · 7 H₂O | 0,222 g |
| NaMoO₄ · 2 H₂O | 0,39 g |
| CuSO₄ · 5 H₂O | 0,079 g |
| Co(NO₃)₂ · 6 H₂O | 49,4 mg |
| Dest. water | 1000 ml |

Because cyanobacteria are photosynthetically active the plates were placed in light. Additionally the media comprised an antibiotic which inhibits the growth of eukaryotic algae (such as green algae).

The table below shows the strains used:

**Table 3: cyanobacterial strains used in the experiments**

| | |
|---|---|
| OT_Ro_Ocya36 | Nostoc sp. |
| OT_Ro_Ocya72 | Leptolyngbya boryana |
| OT_Ro_Ocya105 | Trichormus azollae |
| OT_Ro_Ocya142 | Leptolyngbya sp. |
| OT_Ro_Zollstation_2 | Limnothrix sp. |
| OT_Ro_Ocya39 | Leptolyngbya sp. |
| OT_Ro_Anglerteich_1 | Limnothrix redekei |
| OT_Ro_Ocya2 | Pseudoanabaena sp. |
| OT_Ro_Ocya69 | Synechocystis sp. |

Additionally, the Humboldt University, the Universtiy of Freiburg and the Company Cyano Biotech provided for axenic cyanobacterial reference strains in order for attempting to optimize the AFLP method. See table 4 below. Table 4

Reference strains used for comparison with isolated cyanobacteria.
The phylum was determined using 16S rDNA data.

**Table 4: cyanobacterial reference strains used in the experiments**

| | |
|---|---|
| WT_6803_Rippka | Synechocystis sp. |
| WT_6803_Hagemann | Synechocystis sp. |
| WT_6803_Miche! | Synechocystis sp |
| WT_6803_Forchhammer | Synechocystis sp. |
| WT_6803_Shestakov/HU1 | Synechocystis sp. |
| WT_6803_Shestakov/HU2 | Synechocystis sp. |
| WT_6803_Shestakov/HU3 | Synechocystis sp. |
| WT_6803_Shestakov/HU3_Mut1 | Synechocystis sp. |
| WT_6803_Shestakov/HU3_Mut8 | Synechocystis sp. |
| WT_6803_Giela | Synechocystis sp. |
| WT_6803_Fiedler | Synechocystis sp. |
| WT_6803_Japan | Synechocystis sp. |
| WT_8803_Vermaas | Synechocystis sp. |
| PM_As_9601 | Prochlorochoccus marinus |
| PM_MIT_9312 | Prochlorochoccus marinus |
| PM_MIT_9313 | Prochlorochoccus marinus |
| PM_ss_120 | Prochlorochoccus marinus |
| SynWH_8102 | Synechococcus sp. |

### Isolation of genomic DNA from cyanobacteria

Cells were harvested by centrifugation (4800 rpm, 10 minutes) thereafter the cell pallet was taken up in a volume of TES buffers and shockfrozen at -80 °C for 15 minutes. Upon thawing incubation was performed using lysocyme (5 mg/ml final concentration) at 37 °C in a water bath for 1 hour. Afterwards, proteinase K was added to the solution (100 µglml) as well as SDS (2% final concentration) and the solution was incubated at 60 °C over night. Proteinase K was used for inhibition of DNases and for the degradation of cellular proteins.

Next morning the extraction of proteins was performed using a volume of phenol/chloroform (1:1). This step was repeated upon which an extraction step of DNA was performed using 1 volume of chloroform and thereafter precipitation was done with 0.7 volume isopropanol. The pellet was washed with 70"% ethanol and then air-dried. Then the DNA was dissolved in distilled water. The DNA concentration was measured using a spectral photometer NanoDrop ND 1000 (Thermo Fisher Scientific in Wilmington, Delaware, USA).

16S rDNA sequences were determined from all strains used for the experiments,.

### Method of identifying and/or differentiating cyanophyta

The method of identifying and/or differentiating cyanophyta comprises the following principal steps:
1. restriction of gDNA with restriction enzymes
2. ligation of adaptors
3. pre-amplification
4. selection amplification
5. separation of fragments by capillary electrophoresis
6. Analysis of row data

Initially the restriction of the DNA was done with two different enzymes: a frequently cutting enzyme (frequent cutter) MseI and a seldomly cutting enzyme (rare cutter) EcoRI. Both are type II restriction enzymes and create sticky ends.

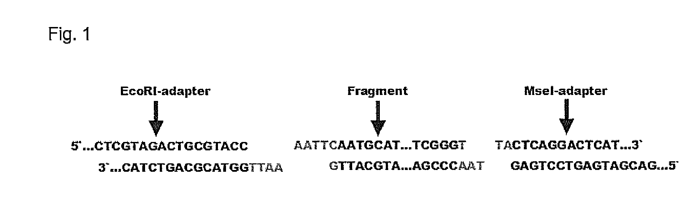

To these sticky ends of the fragments adaptors are now ligated. See Fig. 1.

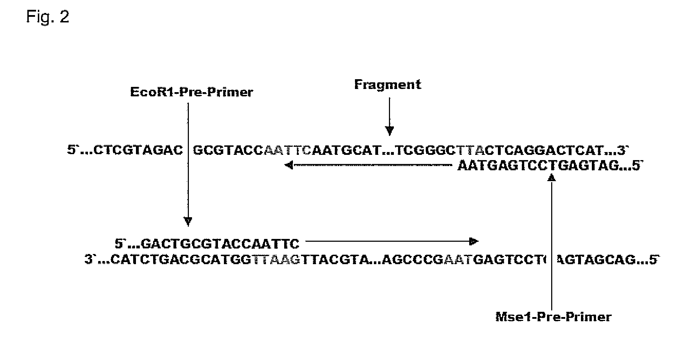

Following adaptor ligation preamplificafiion takes place. The oligonucleotides for preamplification were designed complementary to part of the restriction enzyme recognition sequences as well as to the adaptor sequences. See Fig. 2.
Due to the fact that upon preamplification the number of fragments is very high (several hundred), a selective amplification is performed thereafter. Two selective amplification primers are used which have the same nucleotide sequence as the primers for preamplification. However, additionally they have two selection bases at their respective 3' OH terminal ends. By using these selective primes only a portion of the fragments will be amplified. Using one primer with two degenerated bases at it's 3' OH terminus the number of fragments theoretically will be reduced to 1/16, when using two selective primers with two degenerated bases at their 3' OH terminal end and one MseI-based pre primer the number of fragments will be reduced by 7/8.

The detection of the fragments is possible on different ways and is based upon the the method used for DNA fragment weight separation. If gel electrophoresis is used, the detection of the fragments is carried out by staining the DNA with either methylene blue or DNA intercalation dyes or fragments are labelled radioactively. If capillary electrophoresis is used, the detection of the fragments is performed by labeling.each EcoRI-based selective primer by a fluorescence dye at its 5' end.

In our case we chose fluorescent labels. These labels may be detected using a laser.

### DNA restriction

50-250 ng of genomic DNA was used for digestion. The DNA concentration was measured using a spectral photometer NanoDrop ND 1000 (Thermo Fisher Scientific in Wilmington, Delaware USA). The reaction mixture(10-20 µl) comprised apart from water, BSA, 10 x NEB buffer, each 1 U Mse and 0,5 U Eco1. The digestion was performed for 4 h at 37 °C in a 2720 Thermal Cycler (Applied Biosystems, CA, USA). Thereafter the probes were inactivated at 65 ° for 10 min C in a heating block.

### Ligation of the adaptors

The adaptor oligonucleotides were delivered as single stranded molecules (Sigma Afdrich) and than made doublestranded by thermal treatment and slowly cooling down. The adaptors had the following sequences (this is given in 5'-3' orientation):
EcoRI-adaptor+ CTCGTAGACTGCGTACC (SEQ ID NO. 1)
EcoRI-adaptor- AATTGGTACGCAGTCTAC (SEQ 1D NO. 2)
MseI-adaptor+ GACGATGAGTCCTGAG (SEQ ID NO. 3)
MseI-adaptor- TACTCAGGACTCAT (SEQ ID NO. 4)

The adaptors need to be created from the two oligonucleotides. This is done using the reaction mixture you can see in Table 5 and 6.

**Table 5: Pipeting scheme for construction of MseI-adaptors**

| Muse 1-Adaptor 50 µM | |
|---|---|
| | Volumen [µl] |
| Mse 1-Adapter+ 100 µM | 150 |
| Mse 1-Adapter- 100 µM | 150 |
| Tris-HCL 1 M, pH 8,0 | 3 |
| NaCl 5 M | 3 |
| EDTA 0,5 M, pH 8,0 | 0,6 |

**Table 6: pipet scheme for construction of EcoRI-adaptors**

| EcoR 1-Adaptdr 5 µM | |
|---|---|
| | Volumen [µl] |
| EcoR1-Adapter+ 100 µM | 20 |
| EcoR1-Adapter- 100 µM | 20 |
| Tris-HCL 1 M, pH 8,0 | 4 |
| NaCl 5 M | 4 |
| EDTA 0,5 M, pH 8,0 | 0,8 |
| PCR-H2O | 351,2 |

The adaptor construction is performed at 95 °C for two minutes in a heating block. Thereafter the heating block is turned off and the adaptor reaction mixture is cooled down slowly.

### Libation reaction

For ligation the finalized adaptors and 5 µl of the restriction assay were mixed. A 10 µl ligation assay included water, the ligase and the 10 x ligase buffer, 0.25 µM EcoRI adaptor and 2.5 µM MseI adaptor. The ligation was done for 4 h at 16 °C in the 2720 Thermal Cycler (Applied Biosystems, CA, USA).

### Preamplification

The DNA oligonucleotides for preamplification were obtained from Sigma Aldrich.

### Sequences of the preamplification primers are as follows:

EcoRI-PrePrimer 5'GACTGCGTACCAATTC3' (SEQ ID NO. 5)
MseI-PrePrimer 5'GATGAGTCCTGAGTAA3' (SEQ ID NO. 6)

The preamplification primers were used at a concentration of 0.3 µM. The PCR was performed in a 2720 Thermal Cycler (Applied Biosystems, CA, USA). All reaction mixtures were placed in a 96 well microtiter plate, Each well included 20 µl reaction mix comprising Taq polymerase, 10 x PCR buffer, MgCl₂, dNTP's, the respective primers and 1 µl probe of the adaptor ligation mixture.

The preamplification PCR steps are shown in table 7.

| PCr-Program | | |
|---|---|---|
| | Temperature [°C] | Time [min] |
| Step 1 | 96 | 2 |
| Step 2 | 94 | 0,5 |
| Step 3 | 56 | 1 |
| Step 4 | 72 | 1 |
| Step 5 | Go to step 2 x 20 cycles | |
| Step 6 | 72 | 15 |
| Step 7 | 60 | 15 |
| Step 8 | 4 | ∞ |

### Selective amplification

For selective amplification two selective primers with different combinations of two degenerated bases on their 3' OH terminal end (EcoRI-based) and one preamplification primer (MseI-based) were used (Sigma Aldrich), wherein the EcoRI-based oligonucleotides additionally carry a fluorescent label (see table 8 below).

**Table 7: primer sequences used for selective amplification**

| Selektive primer | 5'-3' sequences | Label |
|---|---|---|
| | | |
| EcaRl-PrimerCA | GACTGCGTACCAATTCCA (SEQ ID NO. 7) | 6FAM |
| EcflRl-PrimerGT | GACTGCGTACCAATTCGT (SEQ ID NO. 8) | HEX |
| MseI-Pre-Primer | GATGAGTCCTGAGTAA (SEQ ID NO. 9) | |

The selective EcoRI primers were used in a concentration of 0.05 µM, whereas the specific MseI primers were used at 0.3 µM.

The PCR was done in a similar fashion as the preamplification. The only difference was that one microliter of probe was used and the primers were the selection primers. The steps of the selective PCR are illustrated in table 9 below:

**Table 8: PCR-program for selection amplification**

| PCR-Program | | |
|---|---|---|
| | Temperature [°C] | Time [min] |
| Step1 | 96 | 1 |
| Step 2 | 95 | 0,5 |
| Step 3 | 60 | 0,5 |
| Step 4 | 72 | 1 |
| Step 5 | Go to step 2 x 25 cycles | |
| Step 6 | 72 | 10 |
| Step 7 | 15 | ∞ |

### Testing the method for robustness and reproducibility

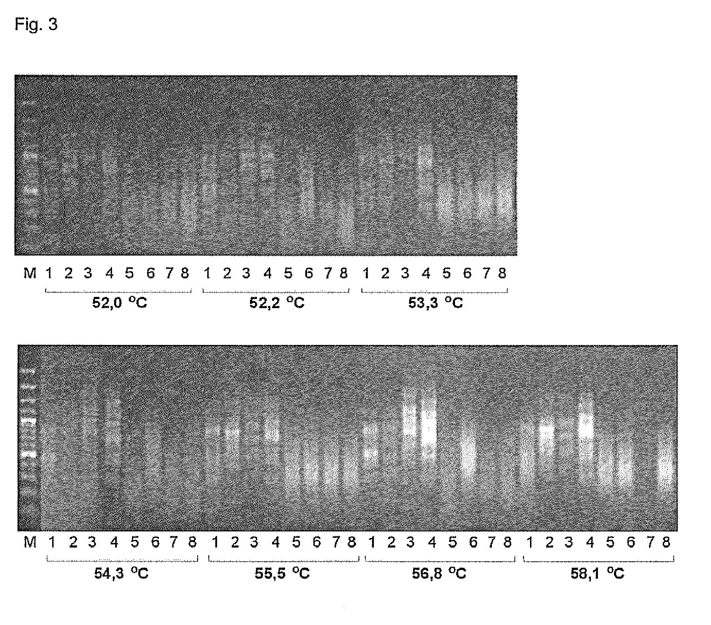

To show the robustness and the reproducibility of the method, we performed the PCR-reactions in a gradient cycler variing the annealing temperatures for the preamplification from 52°C to 58°C and for the selection amplification from 54°C to 66°C. The results of this experiment showed that the chosen temperature regimes have a very low influence on the results. (see Fig. 3.) These experiments demonstrated the stability and robustness of the method.

### Detection of fragments

From a single specific amplification reaction 0.5 µl was mixed with formamide and an internal standard was added. The internal standard was the GeneScan 500 ROX^{Tm} (Applied Biosystems, CA, USA). This consists of 16 fluorescently labeled fragments between 35 und 500 bp. The capillary electrophoresis was done on the automated sequencer 3130 XL (Applied Biosystems, CA, USA) under standard conditions.

### Analysis of fragments

The analysis of fragments was done with GeneMapper Version 4.0 (Applied Biosystems, CA, USA). The program changed the raw data of the automated sequencer into a chromatograms. See Fig. 4. In addition to a chromatogram the program also produced a table with the various peaks and various respective sizes. The table can be exported as a text document. We then used the program "GelCompare II" (Applied Maths, Sint-Martens-Latem, Belgium) to analyze these data files. The program is able to generate graphic views (bending pattern, See Fig. 5 and 6) out off the table data. Further the program is able to compute a dendrogram using the data of the fragments we have produced. See Fig. 5.

### Reamplification and sequencinq of certain fragments

In order to determine what the fragments actually were made up, we re-amplified fragment pools from individual samples and sequenced single fragments cut out of agarose gels after purification.
Various fragments were cut out of the agarose gel. The fragments were cleaned and isolated from the agarose. Sequencing was done using standard methods, whereafter sequence base analysis was performed.

## Claims

1. A method of identifying and/or differentiating cyanophyta comprising the steps of,
a. enzymatically digesting at least a first genomic DNA from a cyanophyta isolate to be identified or differentiated, with at least two different restriction enzymes, into restriction fragments, wherein a first restriction enzyme is a four cutter and a second enzyme is a six cutter,
b. ligating double stranded nucleic acid oligonucleotide adaptor fragments to the nucleic acid ends of the restriction fragments created in step (c)
c. pre-amplifying the fragments using pre-amplification primers which are substantially complementary to at least part of the restriction sites generated in step (a) and substantially complementary to at least part of the double stranded nucleic acid oligonucleotide adaptor fragments ligated in step (b),
d. selectively amplifying the fragments generated after step (c) by using
i. least one pre-amplifying primer and
ii. at least one selection primer which is substantially complementary to at least part of the restriction sites generated in step (a) and substantially complementary to at least part of the double stranded nucleic acid oligonucleotide adaptor fragments ligated in step (b) and which comprises at least two additional nucleotides at its 3'-OH.

2. Method according to claim 1, wherein the step of selective amplification is performed using two or more different selection primers,
a. a first selection primer which is substantially complementary to at least part of the six cutter restriction sites generated in step (a) and substantially complementary to at least part of the double stranded nucleic acid oligonucleotide adaptor fragments ligated in step (b), but comprises two additional nucleotides at the 3'-OH end and,
b. a second selection primers which is substantially complementary to at least part of the six cutter restriction sites generated in step (a) and substantially complementary to at least part of the double stranded nucleic acid oligonucleotide adaptor fragments ligated in step (b), but comprises two additional nucleotides at the 3'-OH end,
c. wherein the first, second or further selection primer do not have the same sequence.

3. Method according to claims 1 or 2, wherein said first selection primer comprises a detection label at it's 5'-end and said second selection primer comprises a detection label at it's 5' end, wherein the first and the second primer are labelled differently.

4. Method according to any of the above claims, wherein the restriction enzymes used in step (a) are EcoRI and MseI.

5. Method according to claim 4, wherein in the selection step, the pre-amplifying primer is an MseI primer and the selection primer is an EcoRI primer.

6. Method according to claim 5, wherein the ratio of the amount of primers EcoRI to MseI is between 1 to 1 and 1 to 50.

7. Method according to any of the above claims, wherein molarity of the EcoRI primer in the reaction is between 0.01 µM and 2.5 µM and the molarity of the MseI primer in the reaction is between 0.06 µM and 1.5 µM.

8. Method according to claims 4 to 7, wherein double stranded nucleic acid oligonucleotide adaptor fragments consist of the following nucleic acid molecules:
EcoRI - adaptor (+) 5'-CTC GTA GAC TGC GTA CC-3' (SEQ ID NO. 1) and
EcoRI - adaptor (-) 5'- AAT TGG TAC GCA GTC TAC -3' (SEQ ID NO. 2) and
MseI - adaptor (+) 5'- GAC GAT GAG TCC TGA G -3' (SEQ ID NO. 3) and
MseI - adaptor (-) 5'- TAC TCA GGA CTC AT -3' (SEQ ID NO. 4).

9. Method according to claim 7, wherein the pre-amplification primers have the following sequences, EcoRI - pre-amplification primer 5'-GACTGCGTACCAATTC-3' (SEQ ID NO. 5) and MseI - pre-amplification primer 5'- GATGAGTCCTGAGTAA-3' (SEQ ID NO. 6)

10. Method according to claims 5 to 9, wherein the molarity of the EcoRI primer in the reaction is between 0.01 µM and 0.25 µM and the molarity of the MseI primer in the reaction is between 0.06 µM and 1.5 µM.

11. Method according to claims 5 to 9, wherein at least two of the selection primers are selected from the following group of primers and wherein these have the following nucleotides at their respective 3'-OH terminal end:
EcoRI-Primer-CA-3'
EcoRI-Primer-GT-3'
EcoRI-Primer-TA-3'
EcoRI-Primer-TT-3'

12. Method according to claims 1 to 11, wherein two selection primers are used and one MseI pre-amplifying primer is used..

13. Method according to claims 5 to 12, wherein the selection primers that may be used have the following sequences:
EcoRI-Primer- 5'- GACTGCGTACCAATTCCA - 3' (SEQ ID NO. 7)
EcoRI-Primer- 5' -.GACTGCGTACCAATTCGT - 3'(SEQ ID NO. 8)
EcoRI-Primer- 5'- GACTGCGTACCAATTCTA - 3'(SEQ ID NO. 9)
EcoRI-Primer-5'- GACTGCGTACCAATTCTT - 3'(SEQ ID NO. 10)

14. Method according to any of the claims, wherein all of the selection primers are labelled differentially.

15. Kit comprising at least 1 pre-amplification primer, 1 labelled selection primer and a sample from Cyanophyta.


SEQUENCE LISTING

<110> Cyano-Biotech GmbH
Cyano Biotech GmbH

<120> METHOD OF IDENTIFYING AND/OR DIFFERENTIATING CYANOPHYTA

<130> P2434 EP BLN

<160> 10

<170> PatentIn version 3.3

<210> 1
<211> 17
<212> DNA
<213> Artificial

<220>
<223> Adaptor

<400> 1
ctcgtagact gcgtacc 17

<210> 2
<211> 18
<212> DNA
<213> Artificial

<220>
<223> adpator (-)

<400> 2
aattggtacg cagtctac 18

<210> 3
<211> 16
<212> DNA
<213> Artificial

<220>
<223> adaptor

<400> 3
gacgatgagt cctgag 16

<210> 4
<211> 14
<212> DNA
<213> Artificial

<220>
<223> adaptor (-)

<400> 4
tactcaggac tcat 14

<210> 5
<211> 16
<212> DNA
<213> Artificial

<220>
<223> primer

<400>  5
gactgcgtac caattc                                                        16


<210>  6
<211>  16
<212>  DNA
<213>  Artificial

<220>
<223>  primer

<400>  6
gatgagtcct gagtaa                                                        16


<210>  7
<211>  18
<212>  DNA
<213>  Artificial

<220>
<223>  selection primer Eco RI 1

<400>  7
gactgcgtac caattcca                                                      18


<210>  8
<211>  18
<212>  DNA
<213>  Artificial

<220>
<223>  primer selection EcoRI nr. 2

<400>  8
gactgcgtac caattcgt                                                      18


<210>  9
<211>  18
<212>  DNA
<213>  Artificial

<220>
<223>  EcoRI selection primer nr. 3

<400>  9
gactgcgtac caattcta                                                      18


<210>  10
<211>  18
<212>  DNA
<213>  Artificial

<220>
<223>  Eco RI selection primer Nr. 4

<400>  10
gactgcgtac caattctt                                                      18